# EUROPÄISCHE PATENTSCHRIFT

(11) **EP 0 622 360 B1**
(45) Veröffentlichungstag und Bekanntmachung des Hinweises auf die Patenterteilung: **04.10.2001**
(21) Anmeldenummer: 94105691.3
(22) Anmeldetag: 13.04.1994
(51) Int. Cl.: C07D 241/44

(54) **Verfahren zur Herstellung von Chinoxalinen**
Process for the preparation of quinoxalines
Procédé de préparation de quinoxalines

(30) Priorität: 26.04.1993 DE 4313586
(43) Veröffentlichungstag der Anmeldung: 02.11.1994
(73) Patentinhaber: BAYER AG, 51368 Leverkusen (DE)
(72) Erfinder: Zarnack, Jens Uwe, Dr., D-25541 Brunsbüttel (DE); Diesselkämper, Bernd, Dr., D-25709 Marne (DE); Lorenz, Wolfgang, Dr., New Matinsville WV 26155 (US); Borchers, Jörg-Michael, D-25724 Neufeld (DE)

(56) Entgegenhaltungen:
- EP-A- 0 050 790
- EP-A- 0 537 540
- DE-A- 3 039 884
- GB-A- 315 451

## Beschreibung

Die Erfindung betrifft ein neues Verfahren zur Herstellung von Chinoxalinen, insbesondere 2,3-Dichlorchinoxalin-6-carbonsäurechlorid.

2,3-Dichlorchinoxalin-6-carbonsäurechlorid ist ein wichtiges Zwischenprodukt zur Herstellung von Reaktivfarbstoffen. Die Chinoxalincarbonsäurechloridgruppierung fungiert in derartigen Farbstoffen als Reaktivgruppe, die chemisch mit der Faser reagiert.

Es sind bereits diverse Herstellungsverfahren für 2,3-Dichlorchinoxalin-6-carbonsäurechlorid bekannt. Gemäß der deutschen Auslegeschrift 1 186 160 kann man z. B. von einer 1,2-Diaminobenzoesäure ausgehen, diese mit Oxalsäure unter Ausbildung eines Chinoxalinringes umsetzen und die freien Hydroxygruppen in Halogensubstituenten abwandeln.

Nachteilig an den bekannten Verfahren ist ihre vergleichsweise unbefriedigende Ausbeute. Der Erfindung lag die Aufgabe zugrunde, ein verbessertes Herstellungsverfahren bereitzustellen.

Die vorliegende Erfindung betrifft ein Verfahren zur Herstellung von Dihalogenchinoxalincarbonsäurehalogeniden der Formel (I) 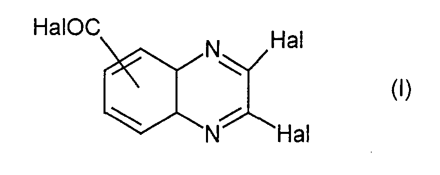 dadurch gekennzeichnet, daß man Diamine der Formel (II) 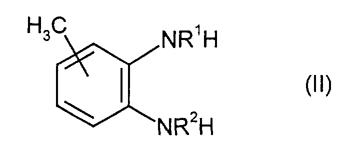 worin
- R¹ und R²: unabhängig voneinander Wasserstoff oder C₁-C₄-Alkyl bedeuten mit Oxalsäure der Formel (III) 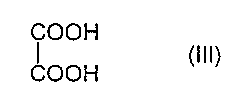
unter Erhalt von Verbindungen der Formel (IV) 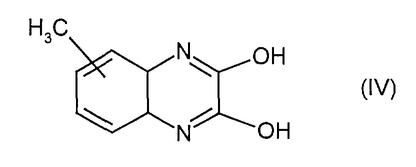 umsetzt und diese thermisch oder photochemisch induziert in Thionylchlorid radikalisch oder ionisch zu Verbindungen der Formel (V) 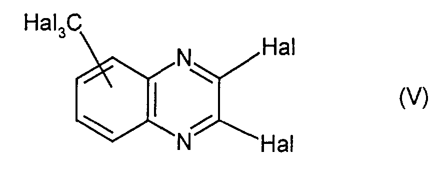 worin
- Hal: für Cl, Br steht,
vorzugsweise mit Chlor in einem Lösungsmittel halogeniert und diese mit Carbonsäure und Metallhalogeniden oxidiert, oder daß man Verbindungen der Formel (V) in Gegenwart eines Lösungsmittels wie chlorierte Aromaten, Tetrachlormethan, Phosphoroxychlorid oder Thionylchlorid und in Gegenwart eines Katalysators wie Pyridin oder N,N-disubstituierte Formamide und in Gegenwart eines Radikalkettenstarters zu Verbindungen der Formel (VI) 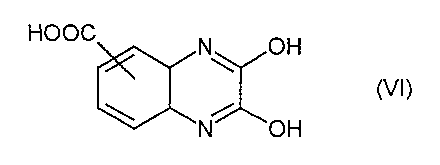 oxidiert und diese schließlich halogeniert.

In einer bevorzugten Ausführungsform steht in Formel (II) der Substituent CH₃ nicht in ortho-Stellung zu den beiden Aminosubstituenten. In einer besonders bevorzugten Ausführungsform bedeuten die Substituenten R¹ und R² Wasserstoff. In einer ganz besonders bevorzugten Ausführungsform wird erfindungsgemäß ausgehend von 3,4-Tolylendiamin als Verbindung II das Endprodukt 2,3-Dichlorchinoxalin-6-carbonsäurechlorid erhalten.

Die Halogenierung findet vorzugsweise mit Licht in Gegenwart eines Lösemittels statt. Die Halogenierung von (V) nach (VI) kann in Gegenwart eines Katalysators durchgeführt werden; geeignete Katalysatoren sind Pyridin und N,N-disubstituierte Formamide, insbesondere Dibutylformamid. Bei thermischer Induzierung der Halogenierung werden zusätzlich handelsübliche Radikalkettenstarter insbesondere Azo-bis-isobutyronitril eingesetzt. Das Trihalogenalkylchinoxalinderivat (V) kann durch Hydrolyse und Austausch der Halogensubstituenten gegen Hydroxylgruppen in die Dihydroxychinoxalincarbonsäure (VI) umgewandelt werden. Dazu wird entweder mit Wasserdampf unter erhöhter Temperatur oder unter Zusatz von Alkali in wäßriger Lösung bei erhöhter Temperatur gearbeitet. Die weitere Umsetzung umfaßt die Halogenierung, insbesondere Chlorierung mit Phosgen, besonders bevorzugt mit Thionylchlorid zur Verbindung I. Diese Halogenierung wird im allgemeinen in Gegenwart eines Katalysators durchgeführt; geeignete Katalysatoren für die Chlorierung sind Pyridin, N,N-disubstituierte Formamide insbesondere Dibutylformamid.

Alternativ kann die Stufe V durch Oxidation mit Carbonsäuren und Metallhalogeniden zum Endprodukt I überführt werden.

Vorzugsweise wird diese Oxidation durchgeführt durch Umsetzung mit Ameisensäure in Gegenwart von Eisen(III)chlorid.

Das neue Herstellungsverfahren basiert auf nur wenigen Grundchemikalien und liefert überraschenderweise eine Gesamtausbeute von über 95 %.

Die erfindungsgemäß hergestellten Chinoxaline können in üblicher Weise zur Herstellung von Reaktivfarbstoffen verwendet werden, insbesondere durch Umsetzung mit aminogruppenhaltigen Farbstoffen oder Farbstoffvorprodukten. Verwiesen wird auf die deutsche Auslegeschrift 1 469 698, DE-A 2 948 292, DE-A 2 948 293, DE-A 2 925 210, DE-A 2 942 964, DE-A 3 207 534, DE-A 3 503 745, DE-A 3 707 549.

### Beispiel

### 1. Reaktionsstufe

180 g Oxalsäure werden in 1 500 ml Wasser gelöst und auf 98 bis 100°C erwärmt. Nach Erreichen der vorgegebenen Temperatur werden vorsichtig 122 g 3,4-Diaminotoluol zur Oxalsäurelösung zugetropft. Das ausgefallene 6-Methyl-2,3-chinoxalindion/-diol wird über eine Nutsche abgesaugt und mit heißem Wasser aminfrei gewaschen. Die Trocknung erfolgt bei 120°C im Heizschrank.
Ausbeute: 173,4 g (0,99 Mol) = 98,5 % der Theorie.

### 2. Reaktionsstufe:

173,4 g 6-Methyl-2,3-chinoxalindion/-diol aus der 1. Reaktionsstufe werden mit 641 g Thionylchlorid und 2 g N,N-Dibutylformamid versetzt. Der Ansatz wird vorsichtig zum Sieden erhitzt. Nach Erreichen der Siedetemperatur wird in das Reaktionsgemisch unter UV-Bestrahlung Cl₂-Gas eingeleitet. Die Einleitgeschwindigkeit soll ca. 40 l/h betragen. Der Reaktionsverlauf wird dünnschichtchromatographisch verfolgt. Nach ca. 5 Stunden ist die Umsetzung beendet. Das unverbrauchte Thionylchlorid kann bei 15 mbar im Wasserstrahlpumpenvakuum und 40°C abdestilliert werden. Das gebildete 2,3-Dichlor-6-trichlormethylchinoxalin bleibt zurück.
Ausbeute: 307,8 g (0,97 Mol) = 98,7 % der Theorie.

### 3. Reaktionsstufe

307,8 g 2,3-Dichlor-6-trichlormethylchinoxalin werden in 2 000 ml siedendem Wasser aufgenommen. Anschließend wird überhitzter Wasserdampf eingeblasen. Zum Abfangen der dabei entstehenden Salzsäure setzt man 220 g Natriumhydroxid zu. Zur Aufarbeitung des Präparates wird der Ansatz zunächst filtriert. Das Filtrat wird dann mit Salzsäure angesäuert, wobei die reine Chinoxalin-6-carbonsäure ausfällt. Diese wird über eine Nutsche abgesaugt und mit heißem Wasser säurefrei gewaschen. Zur Trocknung gibt man die Substanz bei 120°C in den Heizschrank.
Ausbeute: 196,5 g (0,95 Mol) = 99,4 % der Theorie.

### 4. Reaktionsstufe

196,5 g Chinoxalin-6-carbonsäure wird unter Zusatz von 600 g Thionylchlorid und 2 g N,N-Dibutylformamid zum Rückfluß erhitzt. Dabei kommt es unter Bildung von HCl zur Chlorierung der Carboxyl- und Hydroxylgruppen. Anschließend wird der Thionylchloridüberschuß im Wasserstrahlvakuum abdestilliert. Das Endprodukt wird im Exsikkator bei einem Vakuum von 0,5 mbar von noch vorhanden Thionylchloridresten befreit.
Ausbeute: 243,4 g (0,95 Mol) = 98,9 % der Theorie.

## Patentansprüche

1. Verfahren zur Herstellung von Dihalogenchinoxalincarbonsäurehalogeniden der Formel (I) 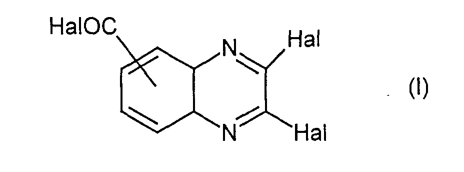 **dadurch gekennzeichnet, daß** man Diamine der Formel (II) 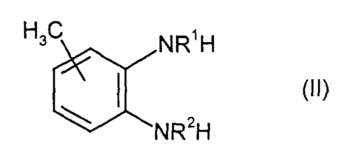 worin
R¹ und R² unabhängig voneinander Wasserstoff oder C₁-C₄-Alkyl bedeuten mit Oxalsäure der Formel (III) 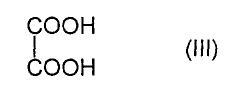
unter Erhalt von Verbindungen der Formel (IV) 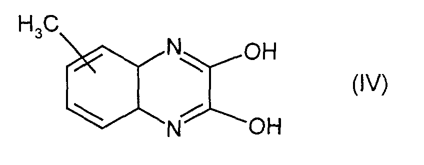 worin
Hal für Cl, Br steht,
halogeniert und diese mit Carbonsäuren und Metallhalogeniden oxidiert, oder **daß** man Verbindungen der Formel (V) in Gegenwart eines Lösungsmittels wie chlorierte Aromaten, Tetrachlormethan, Phosphoroxychlorid oder Thionylchlorid und in Gegenwart eines Katalysators wie Pyridin oder N,N-disubstituierte Formamide und in Gegenwart eines Radikalkettenstarters zu Verbindung der Formel (VI) 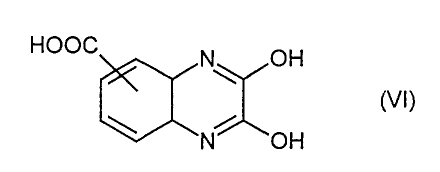 oxidiert und diese schließlich halogeniert.

2. Verfahren nach Anspruch 1, **dadurch gekennzeichnet, daß** der Substituent CH₃ in Verbindung (II) nicht in ortho-Stellung zu den Aminogruppen in der Formel (II) steht.

3. Verfahren nach wenigstens einem der vorhergehenden Ansprüche, **dadurch gekennzeichnet, daß** R¹ und R² Wasserstoff bedeuten.

4. Verfahren nach wenigstens einem der vorhergehenden Ansprüche, **dadurch gekennzeichnet, daß** die Oxidation der Zwischenstufe (V) zu (I) durch Umsatz mit Ameisensäure in Gegenwart eines Katalysators durchgeführt wird.

## Claims

1. Process for preparing dihalogenoquinoxalinecarboxylic halides of the formula (I) 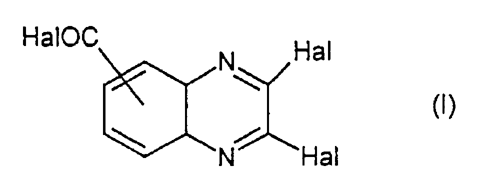 **characterized in that** diamines of the formula (II) 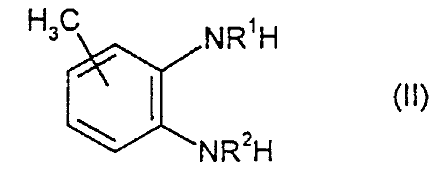 where
R¹ and R² are, independently of one another, hydrogen or C₁-C₄-alkyl, are [lacuna] with oxalic acid of the formula (III) 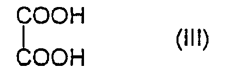
to give compounds of the formula (IV) 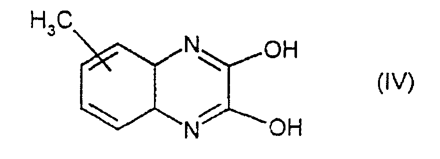 [lacuna] halogenated [lacuna]
where
Hal represents Cl, Br,
and these are oxidized using carboxylic acids and metal halides, or compounds of the formula (V) are oxidized in the presence of a solvent such as chlorinated aromatics, tetrachloromethane, phosphorus oxychloride or thionyl chloride and in the presence of a catalyst such as pyridine or N,N-disubstituted formamides and in the presence of a free-radical initiator to give compounds of the formula (VI) 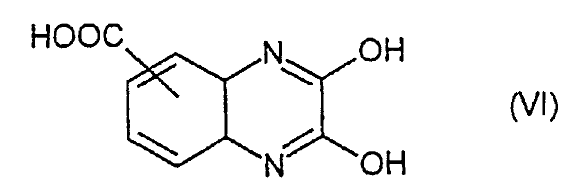 and these are finally halogenated.

2. Process according to Claim 1, **characterized in that** the substituent CH₃ in compound (II) is not in the ortho position relative to the amino groups in formula (II).

3. Process according to at least one of the preceding claims, **characterized in that** R¹ and R² are hydrogen.

4. Process according to at least one of the preceding claims, **characterized in that** the oxidation of the intermediate (V) to (I) is carried out by reaction with formic acid in the presence of a catalyst.

## Revendications

1. Procédé de préparation des halogénures d'acides dihalogénoquinoxalines carboxyliques de formule (I) 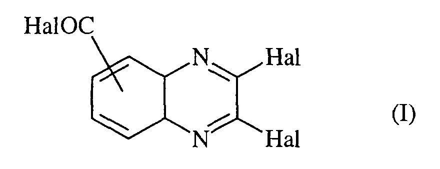 **caractérisé en ce que** l'on halogène des diamines de formule (II) 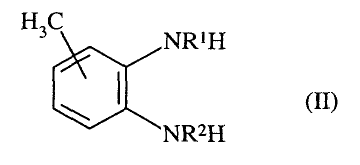 dans laquelle
R¹ et R² représentent chacun, indépendamment l'un de l'autre, l'hydrogène ou un groupe alkyle en C₁-C₄,
avec l'acide oxalique de formule (III) 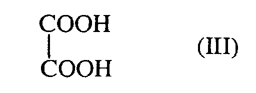
ce qui donne des composés de formule (IV) 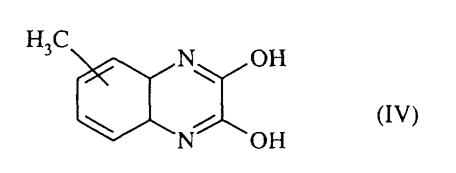 que l'on convertit, avec induction thermique ou photochimique, par halogénation radicalaire ou ionique, en composés de formule (V) 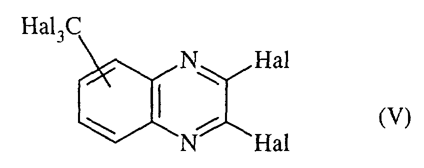 dans laquelle
Hal représente Cl, Br,
que l'on oxyde par des acides carboxyliques et des halogénures métalliques, ou bien, on oxyde des composés de formule (V) en présence d'un solvant tel qu'un hydrocarbure aromatique chloré, le tétrachlorométhane, l'oxychlorure de phosphore ou le chlorure de thionyle et en présence d'un catalyseur tel que la pyridine ou un formamide N,N-disubstitué et en présence d'un inducteur radicalaire, en composé de formule (VI) 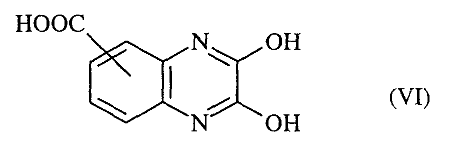 que finalement, on halogène.

2. Procédé selon la revendication 1, **caractérisé en ce que** le substituant CH₃ du composé (II) n'est pas en position ortho des groupes amino de la formule (II).

3. Procédé selon au moins une des revendications qui précèdent, **caractérisé en ce que** R¹ et R² représentent l'hydrogène.

4. Procédé selon au moins une des revendications qui précèdent, **caractérisé en ce que** l'oxydation du produit intermédiaire (V) en (I) est réalisée par réaction avec l'acide formique en présence d'un catalyseur.
